# EUROPÄISCHE PATENTSCHRIFT

(11) **EP 2 911 652 B1**
(45) Veröffentlichungstag und Bekanntmachung des Hinweises auf die Patenterteilung: **21.03.2018**
(21) Anmeldenummer: 13789155.2
(22) Anmeldetag: 04.10.2013
(51) Int. Cl.: C23C 24/08, C23C 24/10, B82Y 40/00, B22F 7/00

(54) **VERFAHREN ZUR HERSTELLUNG EINER NANOPORÖSEN SCHICHT AUF EINEM SUBSTRAT**
METHOD FOR PRODUCING A NANOPOROUS LAYER ON A SUBSTRATE
PROCÉDÉ DE FABRICATION D'UNE COUCHE NANOPOREUSE SUR UN SUBSTRAT

(30) Priorität: 27.10.2012 DE 102012021222
(43) Veröffentlichungstag der Anmeldung: 02.09.2015
(73) Patentinhaber: Forschungszentrum Jülich GmbH, 52425 Jülich (DE)
(72) Erfinder: WOLFRUM, Bernhard, 52074 Aachen (DE); YAKUSHENKO, Alexey, 52068 Aachen (DE); OFFENHÄUSSER, Andreas, B-4731 Eynatten (BE); MAYER, Dirk, 50226 Frechen (DE)
(86) Internationale Anmeldenummer: PCT/DE2013/000567
(87) Internationale Veröffentlichungsnummer: WO 2014/063670

(56) Entgegenhaltungen:
- EP-A1- 2 484 805
- US-A1- 2006 088 567
- US-B2- 7 306 753
- US-B2- 8 137 442
- HONG YOUNG-KYU ET AL: "Controlled two-dimensional distribution of nanoparticles by spin-coating method", APPLIED PHYSICS LETTERS, AMERICAN INSTITUTE OF PHYSICS, US, Bd. 80, Nr. 5, 4. Februar 2002 (2002-02-04), Seiten 844-846, XP012031471, ISSN: 0003-6951, DOI: 10.1063/1.1445811

## Beschreibung

Die Erfindung bezieht sich auf ein Verfahren zur Herstellung einer nanoporösen Schicht auf einem Substrat.

### Stand der Technik

Aus Mukherjee et al. (Mukherjee, B. Viswanath B. and Ravishankar, N. (2010). Functional nanoporous structures by partial sintering of nanorod assemblies, Journal of Physics D: Applied Physics, Volume 43, 455301 (6pp)) ist es bekannt, dass durch abgestimmte Temperaturkontrollen Nanostrukturen gesintert und definierte Poren erzeugt werden können. Die Poren wurden durch partielle Schmelzung und Abrundung durch nachfolgendes Sintern von Nanostab-Kanten erzeugt, die durch anisotropischer Wärmeverteilung heißer waren als der Rest der Nano-Stabs-Strukturen. Dieser Ansatz erlaubt theoretisch die Erhaltung von funktionellen Oberflächen auf den Teilen von Nanostäben, die nicht geschmolzen und gesintert worden sind. Der Schmelzpunkt eines Nanopartikels unterscheidet sich vom Schmelzpunkt des nativen Materials im Bulk. Durch das höhere Oberflächen-zu-Volumen-Verhältnis wird die Schmelztemperatur abhängig von der Partikelgröße stark gesenkt. Dieser Zusammenhang wurde von Buffat und Borel für Nanopartikel aus Gold gezeigt (Buffat, O., Borel, J.-P. (1976). Size effect on the melting temperature of gold particle. Phys. Rev. A, 13, 2287-2298). Es wurde demonstriert, dass Partikel mit einem Durchmesser kleiner als 100 nm bei niedrigeren Temperaturen zu schmelzen beginnen, als dies vom Schmelzpunkt von reinem Gold im bulk bei 1337 K zu erwarten wäre. Der Schmelzpunkt von Partikeln kleiner als 5 nm liegt unter 350 K. Ähnliche Ergebnisse wurden auch für andere Materialien, z. B. für Indium, Zinn, Bismut und Blei von Allen et al. gezeigt (Allen, G.L., Bayles, R.A., Gile, W.W., Jesser, W.A. (1986). Small particle melting of pure metals. Thin Solid Films, 144, 297-308). Der Schmelzpunkt wird auch von der Stabilisierungsschale der Nanopartikel bzw. Lösungsmittel in dem die Partikel gelöst sind beeinflusst (Liang, L.H., Shen, C.M., Du, S.X., Liu, W.M., Xie, X.C., Gao, H.J. (2004). Increase in thermal stability induced by organic coatings on nanoparticles. Physical Review B, 70, 205419 (5 pp)).

Aus Amert et al. (Amert, A.K., Oh, D.-H., Kim, N.-S. (2010). A simulation and experimental study on packing of nanoinks to attain better conductivity. Journal of Applied Physics, 108, 102806 (5pp)) ist bekannt, zwei verschiedene Materialien nacheinander zu sintern um ein besonders dichtes Gefüge auf einem Substrat zu erzeugen.

Zur Herstellung nanoporöser Dünnschichten mit unterschiedlichen Schichtdicken im Bereich von einigen Nanometern bis einigen Mikrometern und Porengrößen von einem Nanometer bis hunderten von Nanometern sind verschiedene Verfahren bekannt. Die Besonderheit dieser Materialien ist das große Verhältnis von Oberfläche zu Volumen. Durch zahlreiche Poren wird die verfügbare aktive oder funktionelle Oberfläche je Volumeneinheit im Vergleich zu planaren Dünnschichten vergrößert.

Dieses Phänomen kann in verschiedenen Bereichen von Vorteil sein. Ein bekanntes Beispiel hierfür sind Immunosensoren für Infektionskrankheiten. Bei diesen Sensoren werden spezifische Bindungsreaktionen zwischen Antikörper und Antigenen ausgenutzt. An die Oberfläche gebundene Antigene können Antikörper, die sich in einem Analyten, z. B. im Blut oder im Speichel befinden, spezifisch binden und umgekehrt. Die durch Bindungsreaktionen erzeugten Signale können z. B. optisch durch Adsorption und Brechungsindex oder elektrochemisch ausgelesen werden, wie z. B. durch Impedanzänderung, Faradaysche oder kapazitive Effekte. Dadurch wird die Konzentration von Antikörpern bestimmt. Nanoporöse Strukturen als Elektroden auf Immunochemischen Sensoren können bei gleichem Sensorvolumen bzw. lateraler Sensorfläche eine Signalverstärkung erzeugen und so die Sensoren sensitiver machen. Je größer die Elektrodenoberfläche ist, desto mehr Antikörper können mit Antigenen reagieren. Zusätzlich können nicht spezifische Bindungssignale von anderen Molekülen, die ein Rauschen im System verursachen und die Detektionschwelle verschlechtern, durch definierte Größen der Poren vermindert werden. Nanoporöse Materialien können zudem in Lab-on-a-Chip Systemen eingesetzt werden. Durch die große Fläche der aktiven Sensorelektroden, kann das System kleiner gemacht werden und die Packungsdichte dieser Sensoren auf einem Chip erhöht werden. Dies trägt dazu bei, dass Lab-on-a-Chip Systeme mit mehreren Sensorelementen für verschiedene Analyten realisiert werden können.

Andere Anwendungen nanoporöser Materialien finden sich in der Energiespeicherung, Katalyse, bei der Membranfertigung, im Gewebeengineering, in der Photonik, Adsorption, Separation und im Drug Delivery. In allen diesen Feldern trägt die große aktive Oberfläche oder bestimmte Porengrößen dazu bei, eine benötige Funktionalität zu erreichen.

Die Verfahren zur Herstellung von nanoporösen Dünnschichten erfolgen in mehreren Schritten mit chemischen, mechanischen oder elektrochemischen Verfahrensschritten. Nachteilig werden dabei eine Vielzahl chemischer Reagentien und Reinraumtechniken angewendet. Eine bekannte Methode zur Herstellung nanoporöser Schichten ist die Anodisierung. Hierzu wird eine Metallschicht, z. B. aus Aluminium, elektrochemisch in einer Säurelösung oxidiert. Durch Selbstorganisation werden nanostrukturierte Filme gebildet.

Ein anderes Verfahren ist die Nanolithographie. Dabei werden Strukturen in vier Schritten realisiert. Als erstes wird eine Dünnschicht des gewünschten Materials durch Chemical Vapor Deposition (CVD), durch Physical Vapor Deposition (PVD), durch Laser Pulsed Deposition (LPD) oder einem äquivalenten Verfahren auf ein Substrat gebracht. Danach wird eine dünne Schicht von lichtempfindlichem Lack aufgebracht. Der Lack wird durch verschiedene lithografische Verfahren strukturiert. Bekannt sind die VIS-Lithographie, die UV-Litographie oder EUV-Lithographie, die E-Beam Lithographie oder die Interference-Lithographie. Der nicht ausgehärtete, das heißt cross-polymerisierte beim positiven Verfahren oder der aufgeweichte, das heißt aufgebrochene chemische Bindungen aufweisende Anteil des Lack beim negativen Verfahren wird durch Waschen entfernt. Danach werden die Poren durch nasse oder trockene Ätzvorgänge in das Material übertragen. Am Ende des Verfahrens wird die Ätzmaske entfernt.

Nachteilig erfordert die Nutzung starker chemischer Reagentien immer auch eine gute Reinigung der gefertigten Dünnschichten, um die Reste toxischer Substanzen zu entfernen. Die Fertigung durch diese Verfahren ist daher aufwändig. Sie muss in mehreren Schritten erfolgen und erfordert bei allen litographischen Verfahren zudem Reinraumtechnologien. Dies treibt die Kosten und die Fabrikationszeiten der produzierten Schichten und Sensoren in die Höhe. Ebenfalls nachteilig sind viele der angezeigten Verfahren nur auf bestimmten Materialien anwendbar, da sie auf bestimmten chemischen Eigenschaften der Substrate angewiesen sind.

US8137442 offenbart ein Verfahren zur Herstellung einer nanoporösen Schicht auf einem Substrat mit einer Auswahl zweier Partikelsorten.

### Aufgabe und Lösung der Erfindung

Aufgabe der Erfindung ist es ein einfaches und kostengünstiges Verfahren zur Herstellung nanoporöser Schichten auf einem Substrat bereit zu stellen, mit dem definiert gleichmäßig verteilte Poren auf einem Substrat hergestellt werden können. Es ist eine weitere Aufgabe der Erfindung eine nanoporöse Schicht bereit zu stellen, die auf einem Substrat gleichmäßig verteilte und definierte Poren aufweist.

Die Aufgabe der Erfindung wird gelöst durch das Verfahren nach Anspruch 1 und durch die nanoporöse Schicht gemäß dem Nebenanspruch. Vorteilhafte Ausgestaltungen ergeben sich aus den hierauf rückbezogenen Patentansprüchen.

Es wurde ein Verfahren auf der Grundlage der physikalischen Parameter der verwendeten Materialien entwickelt. Mindestens zwei Sorten Partikel A und B sowie deren physikalischen Parameter
1. Größe und Form der Partikel A und B sowie
2. Gewichtsverhältnis A:B und
3. Schmelzpunkte der Materialien A und B bei gegebener Partikelform und - Größe (siehe 1.)
werden genutzt um bezüglich der Größe und Form definierte Poren gleichmäßig auf dem Substrat bereit zu stellen.

Folgende Verfahrensschritte werden bei der Herstellung der nanoporösen Schicht auf einem Substrat durchgeführt:
a) Auswahl von mindestens zwei Partikelsorten A und B;
b) Suspendieren beider Partikelsorten A und B in einem Lösungsmittel, wobei die Partikel A und B in einem Gewichtsverhältnis von A:B von 5:1 bis 1000000:1 wt/wt zueinander eingestellt werden
c) Aufbringen der Suspension auf das Substrat;
d) Kontrolliertes Erhitzen der suspendierten Partikel A und B auf dem Substrat durch Heranführen der Temperatur an den Schmelzpunkt S_{mB} der niedriger schmelzenden Partikel, ohne den Schmelzpunkt S_{mA} der bei höherer Temperatur schmelzenden Partikel zu erreichen bzw. ohne die Partikel mit dem höheren Schmelzpunkt zu schmelzen;
e) Überprüfen der porösen Oberfläche.

Zu a) Die mindestens zwei Partikelsorten A und B werden an Hand ihres Unterschieds im Schmelzpunkt ausgewählt. Der Schmelzpunkt ist jeweils abhängig von der Form und der Größe der Partikel A und B sowie von deren chemischer Zusammensetzung. Kleine Partikel B weisen bei derselben Form z. B. einen niedrigeren Schmelzpunkt auf als größere Partikel aus demselben Material. Die Schmelzpunkte der Partikel sind dem Fachmann bekannt oder werden empirisch ermittelt. Der Unterschied zwischen den Schmelzpunkten S_{mA} und S_{mB} der Sorten A und B soll so groß sein, dass in Abhängigkeit von Schritt d) des erfindungsgemäßen Verfahrens ein kontrolliertes Sintern ohne Schmelzen des Materials mit dem höheren Schmelzpunkt möglich ist. In der Regel kann der Unterschied im Schmelzpunkt der mindestens zwei Materialien einige wenige Kelvin betragen, z. B. 20K, 19K, 18K, 17K, 16K, 15K, 14K, 13K, 12K, 11K, 10K, 9K, 8K, 7K, 6K, 5K, 4K, 3K, 2K oder 1K bzw. die entsprechenden Zwischenwerte. Es sollte mindestens 1K Unterschied im Schmelzpunkt der beiden Partikelsorten vorliegen, da ansonsten das Verfahren bei Schritt d) schlecht kontrollierbar ist.

Das Verfahren zur Herstellung nanoporöser Schichten basiert auf einer Mischung verschiedener Partikel, umfassend mindestens zwei Sorten von Nanopartikeln A und B mit unterschiedlichen Schmelzpunkten.

Unter Partikel A und B werden Objekte verstanden, die unterschiedliche Formen und/ oder Größen aufweisen und/ oder aus verschiedenem Material bestehen.

Die Form der Partikel kann identisch sein und z. B. rund, zylindrisch, rechteckig, sternförmig, fadenförmig ausgestaltet sein. Die Form der Partikel A und B kann auch unterschiedlich sein, nämlich rund, zylindrisch, rechteckig, sternförmig, fadenförmig und so weiter.

Dies beeinflusst den Schmelzpunkt. Es ist im Rahmen der Erfindung möglich, dass Partikel A und B unterschiedlicher Form aber im Übrigen aus identischem Material und mehr oder weniger identischer Größe verwendet werden.

Ebenso ist es möglich, Partikel A und B auszuwählen, bei denen die Größe verschiedenartig ist (z. B. Größe A: 100nm und Größe B: 5nm) und im Übrigen die Partikel dieselbe Form (z. B. rund oder sternförmig und so weiter, siehe oben) und auch aus identischem Material (z. B. Gold) bestehen.

Die Größe der Partikel A und B beträgt insbesondere 1nm bis 10µm, insbesondere 1nm bis 100nm, insbesondere 10nm bis 90nm, insbesondere 20nm bis 80nm, insbesondere 25nm bis 75nm, insbesondere 30nm bis 70nm und besonders vorteilhaft 40nm bis 50nm. Die größeren Partikel A sollen insbesondere einen Durchmesser 10 -150nm einnehmen, vorteilhaft 30-130nm. Die kleineren Partikel B sollen insbesondere einen Wert zwischen 1-50nm einnehmen, besonders vorteilhaft sollte eine Partikelgröße von 5-30nm eingestellt werden.

Sowohl die Größe als auch die Form der Partikel A und B beeinflussen den Schmelzpunkt der jeweiligen Partikel. Auch das chemische Material bedingt per se einen Unterschied im Schmelzpunkt der Partikel.

Es ist nicht notwendig verschiedene chemische Materialien für die beiden Partikelsorten zu wählen, sofern der Unterschied im Schmelzpunkt S_{mA} zu S_{mB} anders bedingt wird und ausreichend groß für das anzuwendende Temperaturprogramm ist. Es ist ohne Weiteres denkbar, verschiedene Materialien für die Partikelsorten A und B zu wählen, so dass ein Unterschied im Schmelzpunkt der Partikel A und B allein auf Grund des Materials bedingt wird. Es können also verschiedene Materialien für die Partikel A und B gewählt werden, z. B. verschiedene Metalle, Halbleiter, Keramiken, Polymere jeweils mit oder ohne eine funktionelle Beschichtung der Oberfläche der Partikel. Die Partikel A und B können daher entweder aus einem identischen chemischen Material oder aus verschiedenen chemischem Material bestehen.

Die Nanopartikel A und B und so weiter können aus einem oder mehreren Materialien bestehen und verschiedene innere Strukturen besitzen, nämlich reine Kerne, die mit einem festen oder flüssigen Material gefüllt sind oder leer sind, aus Kernen mit einer oder mehreren Schalen als Funktions- oder Schutzschichten, sowie chemische oder biochemische Terminierung und Funktionalisierung an der Oberfläche der Nanopartikel oder in einer der inneren Schalen.

Die Partikel A und B können optional einen zentralen Kern und weitere Funktionsschalen oder Schutzschalen aus demselben oder unterschiedlichen Materialien umfassen. Die Partikel A und B können somit eine chemische oder biochemische Terminierungen und Funktionalisierungen an der Partikeloberfläche aufweisen. Diese ist für die weitere Anpassung der nanoporösen Schicht und deren Verwendung als Bio- bzw. Immunosensor von besonderer Wichtigkeit.

Die Materialien, die für die Partikelsorten A und B und so weiter besonders genutzt werden, sind die in der Halbleitertechnologie verwendeten Materialien mit guter Leitfähigkeit. Dazu zählen insbesondere aber nicht ausschließlich folgende Materialien: Gold, Silber, Platin, Aluminium, Aluminiumoxid, Silizium, Kupfer, Chrom, Kohlenstoff, Silberchlorid, Titan, Titanoxid, Eisenoxid, Magnesiumoxid, Zinkoxid, Siliziumoxid, Siliziumnitrid, Polyanilin. Es ist wichtig, dass die Partikelsorten A und B und gegebenenfalls weitere Partikelsorten, die die nanoporöse Schicht ausbilden zum Ableiten der gemessenen Signale aus Material mit guter Leitfähigkeit bestehen.

Die Abhängigkeit des Schmelzpunkts der Partikel A und B von der jeweiligen Partikelgröße und Partikelform sowie vom chemischen Material wird ausgenutzt, um die nanoporöse Struktur auf dem Substrat zu erzeugen.

Das Gewichtsverhältnis der Partikel A:B wt/wt ist für die Ausbildung der nanoporösen Schicht und deren Form ebenfalls von Wichtigkeit. Es sollte etwa 5:1 oder größer betragen. Gewichtsverhältnisse zwischen den Partikeln von etwa 5:1 bis etwa 1000000:1 sind ohne weiteres denkbar. Dies liegt daran, dass das Gewicht sphärischer Partikel mit der 3. Potenz des Partikelradius eingeht. Das heißt, ein Gewichtsverhältnis von A:B von 1000000:1 entspricht lediglich einem Verhältnis des Durchmessers von 100:1, unter der Annahme einer gleichen Anzahl sphärischer Partikel des gleichen Materials. Das Oberflächen- zu Volumenverhältnis skaliert bei sphärischen Partikeln mit 1/r, wobei r dem Radius des Partikels entspricht. Bei großen Unterschieden im Gewichtsverhältnis wird das Oberflächen- zu Volumenverhältnis fast ausschließlich durch die größeren Partikel bestimmt. Besonders von Vorteil sind Gewichtsverhältnisse von etwa 10:1 bis etwa 1000:1, insbesondere 20:1 bis etwa 200:1. Bei diesem Verhältnis wird der elektrische Kontakt zwischen großen Partikeln üblicherweise erreicht. Gleichzeitig ist dabei der Anteil der kleinen Partikel noch klein genug, um die Porengröße nicht zu beeinflussen.

Ab einem Gewichtsverhältnis von A:B von ca. 5:1 wt/wt liegen die Gesamtheit der Partikel A als Matrix vor, in die (siehe Schritt b)) die Gesamtheit der Partikel B eingesprenkelt sind. Bei sehr hohen Gewichtsverhältnissen A:B (oberhalb von 1000000:1) kann es dazu kommen, dass kein ausreichender Kontakt innerhalb dieser Matrix erreicht wird.

Gewichtsverhältnisse zwischen 20:1 und 200:1 zwischen den Partikeln sind dabei besonders geeignet. Dabei sollten die Partikel der Sorte A mit dem höheren Schmelzpunkt S_{mA} die Matrix bilden und somit in dem höheren Anteil auf dem Substrat vorliegen. Ansonsten beeinflussen die kleinen Partikel B die Porengröße. Im Extremfall werden die Poren verschlossen und es liegt ein dichter, das heißt nicht poröser Film vor.

Im Wesentlichen bestimmt die Menge und Form der größeren Partikel A mit höherem Schmelzpunkt die Porengröße in der nanoporösen Schicht. Die Porengröße hängt von der Packung der Partikel ab und liegt für sphärische Partikel im Bereich von ca. 0,1*r bis 0,6*r, wobei r dem Radius der Partikel A entspricht.

Durch die Mischungen von Partikeln A und B mit unterschiedlichen Größen und/ oder Formen und/ oder Materialien liegen in der in Schritt d) zu bildenden Schicht Bereiche aus Partikeln mit unterschiedlichen Schmelzpunkten S_{mA} und S_{mB} vor.

Zu b) In Abhängigkeit vom verwendeten Material A und B wird ein für beide Materialien geeignetes Suspensionsmittel ausgewählt und die Materialien A und B in vorgegebenem Gewichtsverhältnis darin gemeinsam suspendiert. Es wird eine homogene Suspension erzeugt, die im Weiteren auf das Substrat aufgebracht wird, um die homogene Verteilung der Partikel A und B auf dem Substrat zu gewährleisten.

In der Suspension befinden sich dann mindestens zwei oder mehr Partikelsorten mit verschiedenen Formen und/ oder Größen und/ oder Materialien.

Als Lösungsmittel bzw. Bindemittel für die Suspension und um die erforderliche Löslichkeit und Viskosität der Suspension zu erreichen, sind verschiedene Lösungsmittel bzw. Bindemittel zu benutzen. Geeignet sind insbesondere Wasser, Ethanol, Methanol, Isopropanol, Hexan, Toluen, Benzen, A-terpineol, Dimethylsulfoxid, Glycerol, Polyvinylpyrrolidon, sowie Elektrolyte und physiologische Puffer, wie PBS, HEPES und andere dem Fachmann gebräuchliche Suspensions- und Lösungsmittel. Das Lösungsmittel wird der Fachmann an Hand der verwendeten Materialien der Partikel A und B entsprechend auswählen.

Zu c) Als Substrat wird jede Oberfläche, die eine gute Adhäsion zu den zu sinternden Nanopartikeln hat, angesehen bzw. ist zu wählen. Das Substrat ist ausreichend stabil für die aufzubringende Suspension und das im Weiteren anzuwendende Sintern. Hier sind insbesondere Metalle, wie z. B. Gold, Silber, Platin, Kupfer und so weiter geeignet. Aber auch Kohlenstoff und deren Varianten, sowie Silizium, Siliziumoxid, Siliziumnitrid und andere Keramiken, Polymere, z. B. Polyethylene, Polypropylene, Polyvinylchloride, Polystyrene, Polycarbonate, Polyimide, Polyester, Surlyn™ und so weiter sind als Substrat besonders geeignet. Auch Papier, welches entweder mit einem Polymer beschichtet ist oder nicht, Textilstoff, welcher entweder mit einem Polymer beschichtet ist oder nicht, Holz, welches mit einem Polymer beschichtet ist oder nicht, ist als Substrat geeignet. Die Substratdicke wird in Abhängigkeit von der Anwendung frei wählbar in einem Bereich von 0,01 µm bis 0,1 m gewählt, je nachdem ob späteres Aufkleben auf andere Auflagen erfolgen soll oder nicht. Verschiedene Techniken können verwendet werden, um die Suspension mit den Partikeln A und B auf das Substrat aufzubringen, nämlich Spincoatingverfahren, Tintenstrahldruckverfahren, Gravurdruckverfahren, Offset-Druckverfahren, Air-Jet-Druckverfahren, sowie mikrofluidische Verfahren. Durch die Einstellung der Verfahrensparameter, wie z. B. der Drehzahl beim Spincoating oder des Tropfenvolumens bei dem Tintenstrahlverfahren, sowie durch eine Viskositätskontrolle bei der Vorbereitung der Suspension, wird die Dicke der deponierten Schicht mit den suspendierten Teilchen darin auf dem Substrat genau kontrolliert. Sie liegt nach dem Aufbringen der suspendierten Partikel A und B vorteilhaft in einem Bereich von etwa 1 nm (Monoschicht aus Nanopartikeln A und B) bis etwa 10 mm.

Optional kann eine Vorheizung des Substrats mit den darauf suspendierten Partikeln erfolgen. Dieser Schritt bewirkt vorteilhaft eine Verdunstung des Suspensions- bzw. Lösungsmittels bei einer Temperatur, die deutlich niedriger ist als der Schmelzpunkt S_{mB} der niedriger schmelzenden Partikel B. Diese optional erfolgende Erhitzung kann bei bestimmten Suspensionen mit Partikeln A und B erforderlich sein, um die Reste von Lösungs- und/ oder Bindemitteln aus der deponierten Schicht auf dem Substrat zu entfernen.

Zu d) Nach dem Aufbringen der Suspension mit Nanopartikeln A und B auf das Substrat werden diese kontrolliert erhitzt bzw. gesintert. Das Sintern kann mittels verschiedener Verfahren durchgeführt werden. Insbesondere geeignet sind Wärme- und Temperaturgradienten, elektromagnetische Strahlung (EM-Strahlung), Bearbeitung der Struktur mit verschiedenen Wellenlängen und auf verschiedener Art, z. B. durch UV-Licht (Wellenlänge = 10 - 400 nm), sichtbares Licht (Wellenlänge = 400 - 800 nm) und Infrarotlicht (Wellenlänge = 0,8 - 300 µm), sowie durch nichtkohärente Lichtquellen und kohärente Lichtquellen (Laserstrahlung) mit kontinuierlicher oder gepulster Form, Mikrowellen-Strahlung (Frequenz = 0,3 - 300 GHz). Auch mit gepulstem elektrischen Strom oder Hochtemperatur-Plasmasintern ist ein kontrolliertes Sintern möglich. Durch kontrollierte Energiezufuhr und durch die Sinterzeit kann die Temperatur genau kontrolliert werden, so dass nur die Nanopartikel mit niedrigerem Schmelzpunkt S_{mB} gesintert werden und verflüssigen. An deren Stelle wird in der Matrix der nicht geschmolzenen Partikel mit dem höheren Schmelzpunkt S_{mA} die poröse Struktur gebildet. Die gebildete Schicht insgesamt ist porös, da die geschmolzenen Partikel mit niedrigem Schmelzpunkt S_{mB} Löcher in der Matrix aus nicht geschmolzenen Partikeln mit höherem Schmelzpunkt S_{mA} bilden, welche bis an das Substrat heranreichen. Es bleiben die Partikel der Sorte A, die erst bei höheren Temperaturen S_{mA} schmelzen also weitgehend intakt. An den Berührungspunkten zu den Partikeln B mit niedrigerer Schmelztemperatur S_{mB} wird durch lokale Schmelzung der Schale der Nanopartikel und durch Schmelzen der Oberfläche die Partikel mit höherem Schmelzpunkt S_{mA} durch Formation von Brücken zusammengeführt.

Durch Nanopartikel mit unterschiedlichem Schmelzpunkt S_{mA} und S_{mB} werden entsprechend der Anzahl verschiedener verwendeter Materialien Dünnschichten aus einem oder mehreren chemischen Materialien aufgebaut. Die Dicke der Dünnschicht sowie die Porengröße der gebildeten nanoporösen Schicht werden durch die Form und die Größe der Nanopartikel A und B und so weiter sowie durch deren Gewichtsverhältnis grundsätzlich festgelegt. Auch das Sinterprogramm beeinflusst die Dicke und die Porengröße. Lange Sinterzeiten an der Temperatur S_{mA} führen zu einer vollständigen Schmelze der Partikel B mit niedrigerem Schmelzpunkt S_{mB}.

Die Eigenschaften von möglichen Funktionsschichten auf den ausgewählten Nanopartikeln A und B, die nicht geschmolzen werden, ändern sich bei einem partiellen Sintern der Partikel selbstverständlich nicht und erhalten somit komplett ihre Funktion. Temperaturempfindliche Partikelkerne, z. B. mit einem Kern aus Gold, die mit Antikörpern funktionalisiert werden und Sintermethoden benutzen. Durch Wärmeeintrag mittels Mikrowellen-Strahlung werden nur die Kerne zum Schmelzen gebracht. Die organischen Antikörper absorbieren im Gegensatz zum Gold kaum Energie in diesem Wellenlängenbereich. Die elektrischen Eigenschaften von dem geformten porösen Material können sich durch eine geeignete Wahl der Nanopartikel verändern lassen.

Der Wärmeeintrag kann auch durch Temperaturrampen in einem normalen oder Konvektionsofen oder in einem Induktionsofen erfolgen. Weiterhin kann er durch elektromagnetische Strahlung (EM-Strahlung), Bearbeitung mit verschiedenen Wellenlängen (UV- Licht = 10 - 400 nm), sichtbares Licht (Wellenlänge = 400 - 800 nm) und Infrarot- Licht (Wellenlänge = 0,8 - 300 µm) mittels nichtkohärenten Lichtquellen oder Laserstrahlung mit kontinuierlicher und gepulster Form, durch Mikrowellen-Strahlung (Frequenz = 0,3 - 300 GHz), durch elektrischen Strom, durch Hochtemperatur- Plasmasintern oder durch heiße, inerte oder aktive (führt zur Reaktion an der Nanopartikeloberfläche) Lösung mit gewünschter Temperatur erfolgen.

Die Zeit, die Leistung und die genaue Art des Sinterverfahrens wird abhängig von der Nanopartikelart (Material, Nanopartikelgröße und Form, Anzahl der verschiedenen Nanopartikel in der Suspension) und deren Eigenschaften, das heißt insbesondere den Schmelzpunkten, eingestellt. Dadurch wird die gewünschte Porengröße und Form definiert eingestellt und eine gleichmäßige Verteilung der Poren erzielt. Die Funktionalität an der Oberfläche der Nanopartikel oder im Kern von nicht gesinterten Nanopartikeln wird beibehalten.

Zu e) Die gebildete nanoporöse Schicht wird z. B. mittels Rasterelektronenmikroskopie oder anderer optischer Verfahren überprüft.

Das Verfahren weist Vorteile gegenüber den bekannten Verfahren auf:
- Während des Verfahrens wird kein Material verloren, da keine Ätzschritte und Waschschritte (Lift Off etc.) während des Verfahrens angewendet werden.
- Größe und Form der Nanoporen können direkt durch die Auswahl der Größe und Art der Nanopartikel kontrolliert werden.
- Nanoporen aus zwei oder mehr verschiedenen Materialien (Alloys) können durch die Auswahl von Nanopartikeln aus unterschiedlichen Materialien hergestellt werden.
- Die nanoporöse Schicht kann auf jegliches Substrat aufgebracht werden, weil die Bindung der Nanopartikel zum Substrat durch Lösungsmittel und Schale der Nanopartikel justiert werden kann.
- Das Verfahren lässt sich durch druckbare Tinten in "printed electronics"-Verfahren integrieren. Das heißt, es lassen sich Anwendungen basierend auf dieser Technik sehr flexibel und kostengünstig herstellen.

Alle Schritte können mehrmals durchgeführt werden, um Dünnschichten mit verschiedenen Dicken oder Funktionalitäten zu erzeugen.

Die auf diese Weise hergestellten nanoporösen Schichten zeigen Unterschiede zu aus dem Stand der Technik bekannten nanoporösen Strukturen auf:
- Die Strukturen haben eine Verteilung bzgl. Größe und Form, das heißt es existieren Nanoporen bzw. Nanokanäle mit definierten Größen und Formen. Diese Verteilung der Porengröße ist breiter, als eine die z. B durch präzise lithographische Verfahren erreicht wird.
- Größe und Form der Nanoporen werden direkt durch die Auswahl der Größe und Art der Nanopartikel kontrolliert.
- Nanoporen aus zwei oder mehr verschiedenen Materialien (Alloys) können hergestellt werden durch die Auswahl von Nanopartikeln aus unterschiedlichen Materialien.

Die Partikel A sind durch und über die geschmolzenen Partikel B elektrisch miteinander verbunden, so dass eine nanoporöse Elektrode gebildet wird.

Das erfindungsgemäße Verfahren ist hierauf noch nicht beschränkt. Vielmehr ist es ein weiteres Ziel der vorliegenden Erfindung, das erfindungsgemäße Verfahren nach den Schritten a) - e) einer weiteren Modifizierung zu unterziehen. Zur Vermittlung bestimmter Funktionalitäten können die hergestellten Dünnschichten bzw. Elektroden nasschemisch oder durch andere Verfahren, wie z. B. durch Plasmaanwendungen, durch Elektrolyseverfahren und so weiter modifiziert werden, um gewünschte chemische, biologische oder physikalische Eigenschaften von der deponierten und gesinterten porösen Dünnschicht zu erstellen.

Eine Beladung der nanoporösen Schicht mit Antigenen oder Antikörpern kann insbesondere durchgeführt werden um Immunosensoren bereit zu stellen.

### Ausführungsbeispiel

Im Weiteren wird die Erfindung an Hand eines Ausführungsbeispiels näher erläutert ohne, dass es hierdurch zu einer Einschränkung der Erfindung kommen soll.

Es zeigen:
- Figur 1: Schematische Darstellung des erfindungsgemäßen Verfahrens.
- Figur 2: Rasterelektronenmikroskopische Aufnahme von einer Dünnschicht aus Goldnanopartikeln verschiedener Größe.

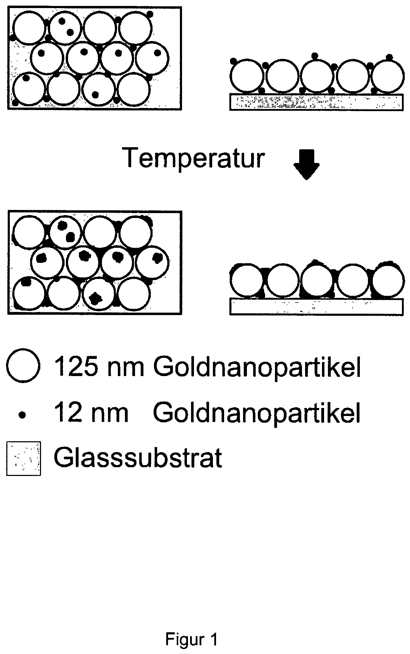

In Figur 1 ist in Aufsicht links im Bild die auf das Substrat aufgetragene Suspension dargestellt.

Zwei verschiedene Partikelsorten A und B werden für die Herstellung einer nanoporösen Schicht ausgewählt. Eine erste Sorte von Nanopartikeln B sind runde homogene Goldnanopartikel mit einem durchschnittlichen Durchmesser von 10 nm und einer Schutzschicht aus Oleylamin. Die Nanopartikel B werden durch ein Verfahren nach Kisner et al. hergestellt (Kisner, A.; Lenk, S.; Mayer, D.; Mourzina, Y.; Offenhäusser, A. J. Phys. Chem. C 2009, 113, 20143-20147). Eine zweite Sorte von Nanopartikeln A sind runde homogene Goldnanopartikel mit einem durchschnittlichen Durchmesser von 125 nm und einer Schutzschicht aus alkanischem Amin. Die Goldpartikel wurden gekauft (http://www.nanopartz.com/ (part # E11-125).

Eine homogene Mischung von diesen zwei verschiedenen Arten von Nanopartikeln A und B wird vorbereitet. Toluol wird als Lösungsmittel in dieser Mischung verwendet. Der prozentuelle Gewichtsanteil von kleineren Nanopartikeln B (10 nm) mit dem empirisch bestimmten Schmelzpunkt S_{mB} von 280° C in der Mischung beträgt 0,24%. Der prozentuelle Gewichtsanteil von größeren Nanopartikeln A (125 nm) mit dem empirisch bestimmten Schmelzpunkt S_{mA} von 350° C in der Mischung beträgt 5,67%. Das Gewichtsverhältnis zwischen den beiden Partikelsorten A:B beträgt etwa 23:1 wt/wt.

Ein Tropfen mit einem Volumen von 1 Mikroliter wird mit einer Laborpipette auf ein Glassubstrat (Objektträger von ThermoScientific, Dimensionen: 76 x 26 x 1 (Höhe) mm) aufgebracht. Das Lösungsmittel wird bei Raumtemperatur innerhalb von 5 Minuten verdampft.

Das Glassubstrat mit dem angetrockneten Rückstand aus der Nanopartikelmischung wird auf der Heizplatte bei 300° C für 5 Minuten erhitzt, um das Sintern von Nanopartikeln B zu verursachen ohne die Partikel A zu schmelzen. Anschließend wird das Ergebnis überprüft.

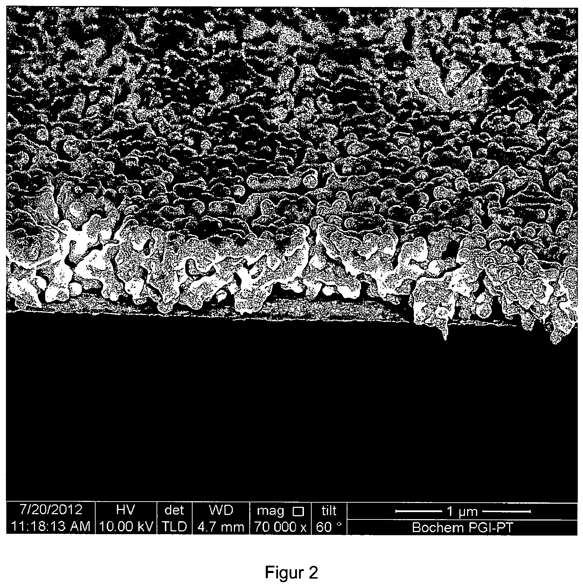

Figur 2 zeigt ein REM-Bild der nanoporösen Schicht. Gleichmäßig verteilte laterale Poren und vertikale Poren bilden eine dichte schwammartige Struktur. Es sind die bis auf die Substrat-Oberfläche reichenden Poren erkennbar. Durch das Sintern ändert sich der Rückstand zu einer nanoporösen Schicht mit unterschiedlicher Porengröße im Bereich von 10 bis 60 nm. Die Porengröße liegt in dem zu erwartenden Bereich. Im Falle einer idealisierten hexagonal dichtesten Kugelpackung werden Zwischenraumgrößen von 28 nm und 52 nm (0,225*r für tetrahedralische Zwischenbereiche und 0,414*r für oktahedralische Zwischenbereiche) erwartet. Über die Partikelgröße wird entsprechend die Größe der Poren in der nanoporösen Schicht definiert eingestellt.

Die gemäß Verfahren der Figur 1 hergestellte nanoporöse Schicht wird im Weiteren funktionalisiert und in einer ersten Alternative als Immunosensor benutzt. Dafür wird die hergestellte nanoporöse Elektrode durch Thiol-Bindung oder durch eine Amin-Bindung oder durch physikalische Verfahren, wie z. B. Physisorption mit spezifischen Antigenen oder Antikörpern, funktionalisiert. Die Funktionalisierung kann vor dem Sintern oder nach dem Sintern in Schritt d) erfolgen. Zusätzlich wird die gebildete Elektrode elektrisch kontaktiert, um das Auslesen elektrischer Signale nach der Bindung eines Analyten an die funktionalisierte Schicht zu ermöglichen. Dies wird durch ein Tintendruckverfahren mit leitenden Goldnanopartikeln erreicht.

Für die immunosensitive Detektion wird ein Tropfen Blut oder Serum auf die Elektrode aufgebracht. Die Änderung der Kapazität bzw. des Widerstands oder der Impedanz des Sensors wird über elektrochemische Impedanzspektroskopie ausgelesen und interpretiert.

Die gemäß der Figur 1 hergestellte nanoporöse Schicht wird im Weiteren funktionalisiert und in einer zweiten Alternative als Immunosensor gegen Malaria benutzt. Dafür wird die hergestellte nanoporöse Elektrode durch weitere Schritte modifiziert.

Die hergestellte nanoporöse Schicht wird mit einer Leiterbahn aus Silberkleber (Epo-Tek H20E-PFC) kontaktiert, um eine Schnittstelle zum elektrischen Kontaktieren zu erzeugen. Danach wird der Sensor bei 150° C für 1 Stunde getrocknet.

Ein Glasring (Durchmesser 5 mm, Höhe 1 mm) wird auf der nanoporösen Struktur mit Polydimethylsiloxane (PDMS, Sylgard® 184) geklebt, um ein Reservoir zu formen. Danach wird der Sensor bei 150° C für 1 Stunde getrocknet.

Sodann werden Antigene gegen Malaria, nämlich Histidine Rich Protein 2 (HRP-2) auf der nanoporösen Schicht durch Physisorption immobilisiert. Im Detail werden hierfür 50 µL einer Lösung von HRP-2 Antigenen (Konzentration = 10 ng/µl) im PBS-Puffer auf der nanoporösen Schicht aufgebracht und bei 37° C für 1 Stunde im Inkubator bei 100% Feuchtigkeit inkubiert. Danach wird der Sensor dreimal mit deionisiertem Wasser gespült, um nicht gebundene Antigene zu entfernen.

Danach werden die nicht besetzten Stellen auf dem Sensor mit Bovine Serum Albumin (BSA) blockiert. Im Detail werden 50 µL 3% Lösung von BSA in PBS-Puffer auf dem Sensor aufgebracht und bei 37° C für 1 Stunde im Inkubator bei 100% Feuchtigkeit inkubiert. Danach wird der Sensor erneut dreimal mit deionisiertem Wasser gespült, um nicht gebundenes BSA zu entfernen. Danach wird der Sensor getrocknet.

Um die Messung zur Detektion von Malaria-Antikörpern im Blut zu realisieren, benötigt man einen elektronischen Messverstärker (VSP-300 der Firma BioLogic, PGSTAT von Autolab, CHI832B von CH Instruments), um die Impedanzspektroskopiemessung durchzuführen. Zusätzlich wird eine Referenzelektrode, z. B. Ag/AgCl benötigt, um den elektrischen Kreis zu schließen.

Ein Tropfen Blut, verdünnt oder auch nicht, mit Volumen von 1 bis 50 µL wird auf den Sensor gebracht. Elektrochemische Impedanzmessung durch den geschlossenen Kreis von modifizierten nanoporösen Elektroden und Ag/AgCl Referenzelektroden wird durchgeführt und zwar im Frequenzbereich von 100 Hz bis 100 kHz. Als Referenzmessung wird ein Tropfen Blut (1 bis 50 µL) vom gleichen Patienten auf einem Sensor gemessen, der genauso hergestellt wurde, außer Schritt 3, d.h. ohne spezifische HRP-2 Antigengen gegen Malaria.

Das Ergebnis von dem Referenzsensor (ohne HRP-2 Antigene) wird mit dem spezifisch modifiziertem Sensor (mit HRP-2 Antigenen) verglichen und, falls es einen Unterschied zwischen beiden gibt, wird ausgeschlossen, dass der Test für HRP-2 Malaria-Antigene positiv war. Es können Mikrosensoren gegen verschiedene Antigene auf einem Substrat realisiert werden.

Es werden weitere Ausführungsbeispiele vorgestellt.

Ausführungsbeispiel mit verschiedenen Nanopartikelgrößen:
Zwei verschiedene Arten von Nanopartikeln werden für die Herstellung einer nanoporösen Schicht ausgewählt. Eine Art von Nanopartikeln sind runde homogene Goldnanopartikel mit einem durchschnittlichen Durchmesser von 10 nm und einer Schutzschicht aus Oleylamin, selbst hergestellt durch ein Verfahren wie beschrieben von Kisner et al¹. Die zweite Art von Nanopartikeln sind runde homogene Goldnanopartikel mit einem durchschnittlichen Durchmesser von entweder 40/60/80/100 oder 125 nm und einer Schutzschicht aus alkanischem Amin gekauft von http://www.nanopartz.com/ (part # E11).

Eine Mischung von diesen zwei verschieden Arten von Nanopartikeln wird vorbereitet. Toluol wird als Lösungsmittel in dieser Mischung benutzt. Der prozentuelle Gewichtsanteil von kleineren Nanopartikeln (10 nm) mit dem empirisch bestimmten Schmelzpunkt Sₘ₁ von 280° C in der Mischung ist 0,24%. Der prozentuelle Gewichtsanteil von größeren Nanopartikeln (40-125 nm) mit dem empirisch bestimmten Schmelzpunkt Sₘ₂ von 320-350° C in der Mischung ist 5,67%.
Ein Tropfen mit dem Volumen von 1 Mikroliter wird auf das Glassubstrat (Objektträger von ThermoScientific, Dimensionen: 76 x 26 x 1 (Höhe) mm) mit einer Laborpipette gebracht. Das Lösungsmittel wird bei Raumtemperatur innerhalb von 5 Minuten verdampft.
Das Glassubstrat mit dem ausgetrockneten Tropfen der Nanopartikelmischung wird auf der Heizplatte bei 300° C für 5 Minuten erhitzt um das Sintern von Nanopartikeln zu verursachen.
Die geformte nanoporöse Schicht wird mit Rasterelektronmikroskopie überprüft. Durch das Sintern ändert sich der getrocknete Tropfen zu einer nanoporösen Schicht mit unterschiedlicher Porengröße in Bereich von 20 bis 100 nm (Durchschnitt von ca. 50 nm, abhängig von der ausgewählter Art des zweiten Nanopartikel).

Diese nanoporöse Schicht kann weiter für die Anwendung als Immunosensor mit elektrochemischer Impedanzspektroskopie als Ausleseverfahren benutzt werden.

Ausführungsbeispiel mit verschiedenen Nanopartikelformen:
Zwei verschiedene Arten von Nanopartikeln werden für die Herstellung einer nanoporösen Schicht ausgewählt. Eine Art von Nanopartikeln sind runde homogene Goldnanopartikel mit einem durchschnittlichen Durchmesser von 10 nm und einer Schutzschicht aus Oleylamin, selbst hergestellt durch ein Verfahren wie beschrieben von Kisner et al.¹. Die zweite Art von Nanopartikeln sind stäbchenförmige Goldnanopartikel mit einem durchschnittlichen Durchmesser von entweder 25/40/ oder 50 nm und einer Länge von 100/115/145/165/250±10 nm, und einer Schutzschicht aus alkanischem Amin (gekauft von http://www.nanopartz.com/ (part # A12)).

Eine Mischung von diesen zwei verschieden Arten von Nanopartikeln wird vorbereitet. Toluol wird als Lösungsmittel in dieser Mischung benutzt. Der prozentuelle Gewichtsanteil von runden Nanopartikeln (10 nm) mit dem empirisch bestimmten Schmelzpunkt Sₘ₁ von 280° C in der Mischung ist 0,25%. Der prozentuelle Gewichtsanteil von stäbchenförmigen Nanopartikeln (mit 25/40/ oder 50 nm Durchmesser und 100/115/145/165/250±10 nm Länge) mit dem empirisch bestimmten Schmelzpunkt Sₘ₂ von 320-350° C in der Mischung ist 6%.
Ein Tropfen mit dem Volumen von 1 Mikroliter wird auf das Glassubstrat (Objekträger von ThermoScientific, Dimensionen: 76 x 26 x 1 (Höhe) mm) mit einer Laborpipette gebracht. Das Lösungsmittel wird bei Raumtemperatur innerhalb von 5 Minuten verdampft.
Das Glassubstrat mit dem ausgetrockneten Tropfen der Nanopartikelmischung wird auf der Heizplatte bei 300° C für 5 Minuten erhitzt um das Sintern von Nanopartikeln zu verursachen.
Die geformte nanoporöse Schicht wird mit Rasterelektronmikroskopie überprüft. Durch das Sintern ändert sich der getrocknete Tropfen zu einer nanoporösen Schicht mit unterschiedlicher Porengröße in Bereich von 20 bis 100 nm (Durchschnitt von ca. 50 nm, abhängig von der ausgewählten Art des zweiten Nanopartikel). Zusätzlich sind dank ihrer Form die Nanoporen aus stäbchenförmigen Nanopartikel anisotropisch, d.h. es gibt gewisse Asymetrie in den Poren.

Solche anisotropische nanoporöse Schicht kann in Surface Enhanced Raman Scattering (SERS) benutzt werden um, wie es aus Literatur bekannt, Plasmonresonanzen mit bestimmter spektralen Eigenschaften zu erzeugen.

Weitere Schritte zur Herstellung eines funktionellen immunologischen SERS-Sensor:
Ein Glasring (Durchmesser 5 mm, Höhe 1 mm) wird auf der nanoporösen Struktur mit Polydimethylsiloxan (PDMS, Sylgard® 184) geklebt um ein Reservoir zu formen. Danach wird der Sensor bei 150° C für 1 Stunde getrocknet.

Als nächstes folgt ein optionaler Schritt zur Immobilisierung von Antigenen gegen Malaria. Hierfür werden das Histidine Rich Protein 2 (HRP-2) auf der nanoporösen Schicht durch physikalische Adsorption immobilisiert. Im Detail: 50 µL einer Lösung von HRP-2 Antigenen (Konzentration = 10 ng/µl) im PBS-Puffer (phosphate buffered saline) wird auf der nanoporösen Schicht aufgebracht und bei 37° C für 1 Stunde inkubiert (im Inkubator bei 100% Luftfeuchtigkeit). Danach wird der Sensor 3-mal mit deionisiertem Wasser gespült um nicht gebundene Antigene zu entfernen. Als Alternative können auch die cDNA-Fragmente zu den entsprechenden Proteinen (HRP-2) immobilisiert werden.
Als nächster Schritt werden die nicht besetzten Stellen auf dem Sensor mit Bovine Serum Albumin (BSA) blockiert. Im Detail, 50 µL einer 3% Lösung von BSA in PBS-Puffer werden auf den Sensor gebracht und bei 37° C für 1 Stunde im Inkubator bei 100% Luftfeuchtigkeit inkubiert. Danach wird der Sensor 3-mal mit deionisiertem Wasser gespült um nicht gebundenes BSA zu entfernen. Anschließend wird der Sensor getrocknet und bis zur Anwendung aufbewahrt.
Um die Messung zur Detektion von Malaria-Antikörper/cDNA im Blut zu realisieren, braucht man ein SERS Gerät (z.B. PSA von Real Time Analyzers, Inc.).
Ein Tropfen Blut, evtl. verdünnt, mit einem Volumen von 1 bis 50 µL wird auf den Sensor gebracht. Für den Fall, in dem cDNA anstatt Antikörper immobilisiert ist, müssen die Malaria-Parasiten, die sich im Blut befinden, zunächst lysiert werden. Für diesen Schritt kann, z.B. TritonX-100 verwendet werden (5% vom Blut-Volumen).
Das Blut-Lysat wird dann auf dem Sensor gebracht, die weiteren Schritte sind analog zu der Prozedur mit immobolisierten Antikörpern.
Nach der Inkubationszeit von 15 min wird der Sensor 3-mal mit PBS und 3 Mal mit destilliertem Wasser gewaschen.
Der Sensor wird mit einer SERS-Geräte internen Lichtquelle angeregt und das SERS-Spektrum wird aufgenommen. Als Referenzmessung wird ein Tropfen Blut (1 bis 50 µL, inkl. zusätzlicher Schritte für die Lysierung im Fall der cDNA-Detektion) vom gleichen Patienten auf einem Referenz-Sensor gemessen. Dieser Referenz-Sensor wir analog zum eigentlichen Sensor hergestellt nur dass Schritt 2 ausgelassen wird. Das heißt, es ist ein Sensor ohne spezifische HRP-2 Antigengen/cDNA gegen Malaria gebildet.

Das Spektrum von dem Referenzsensor (ohne HRP-2 Antigene/cDNA) wird mit dem spezifisch-modifiziertem Sensor (mit HRP-2 Antigenen) verglichen. Eine unterschiedliche Antwort der beiden Sensoren gibt an, dass der Test für HRP-2 Malaria-Antigene positiv war.

Weitere Schritte zur Herstellung des funktionellen chemischen SERS-Sensor:
Ein Glasring (Durchmesser 5 mm, Höhe 1 mm) wird auf der nanoporösen Struktur mit Polydimethylsiloxane (PDMS, Sylgard® 184) geklebt um ein Reservoir zu formen. Danach wird der Sensor bei 150° C für 1 Stunde getrocknet.
1 bis 50 µL von der Lösung mit der chemischen Substanz, die getestet werden muss (z.B. Ethanol mit Reste von giftigem Methanol), werden auf den Chip gebracht.
Der Sensor wird in einem SERS-Gerät mit interner Lichtquelle angeregt und das SERS-Spektrum wird aufgenommen. Als Referenzmessungen und für Kalibrierungszwecke wird eine Reihe von Lösungen mit gleichen Komponenten (Ethanol/Methanol) und definierten Konzentrationen auf dem Chip gebracht und getestet. Durch die vorher erstellte Kalibrierungskurve aus den Referenz-Spektren wird die Konzentration von Methanol in Ethanol in der gemessenen Probe auf Basis des aufgenommenen SERS-Spektrums bestimmt.

Durch die Anpassung der Porengröße und Anisotropie der leitfähigen nanoporösen Schicht ändern sich die Eigenschaften der Oberflächen-Plasmonenresonanz (surface plasmon resonance, SPR) und damit die optischen Eigenschaften der nanoporösen Schicht. Dies ermöglicht die Anpassung von der Plasmonresonanzfrequenz und dadurch verbesserte SERS-Signale von den detektierten Molekülen (Antikörper/cDNA/chemische Molekülen).
(1) Kisner, A.; Lenk, S.; Mayer, D.; Mourzina, Y.; Offenhäusser, A. J. Phys. Chem. C 2009, 113, 20143-20147.
(2) Hsu, S.-W.; On, K.; Tao, A. R. J. Am. Chem. Soc. 2011, 133, 19072-19075.

Ausführungsbeispiel mit verschiedener chemischer Zusammensetzung der Nanopartikel:
Zwei verschiedene Arten von Nanopartikeln werden für die Herstellung einer nanoporösen Schicht ausgewählt. Eine Art von Nanopartikeln sind runde homogene leitende Goldnanopartikel mit einem durchschnittlichen Durchmesser von 10 nm und einer Schutzschicht aus Oleylamin, selbst hergestellt durch ein Verfahren wie beschrieben von Kisner et al.¹. Die zweite Art von Nanopartikeln sind runde homogene halbleitende Siliziumnanopartikel mit einem durchschnittlichen Durchmesser von 30 nm gekauft von http://www.meliorum.com (Produkt #09820).

Eine Mischung von diesen zwei verschieden Arten von Nanopartikeln wird vorbereitet. Toluol wird als Lösungsmittel in dieser Mischung benutzt. Der prozentuelle Gewichtsanteil von runden Goldnanopartikeln (10 nm) mit dem empirisch bestimmten Schmelzpunkt Sₘ₁ von 280° C in der Mischung ist 0,1%. Der prozentuelle Gewichtsanteil von runden Siliziumnanopartikeln (30 nm) mit dem Schmelzpunkt Sₘ₂ von über 400° C in der Mischung ist 10%.

Ein Tropfen mit dem Volumen von 1 Mikroliter wird auf das Glassubstrat (Objekträger von ThermoScientific, Dimensionen: 76 x 26 x 1 (Höhe) mm) mit einer Laborpipette gebracht. Das Lösungsmittel wird bei Raumtemperatur innerhalb von 5 Minuten verdampft.
Das Glassubstrat mit dem ausgetrockneten Tropfen der Nanopartikelmischung wird auf der Heizplatte bei 300° C für 5 Minuten erhitzt um das Sintern von Nanopartikel zu verursachen.
Die geformte nanoporöse Schicht wird mit Rasterelektronmikroskopie überprüft. Durch das Sintern ändert sich der getrocknete Tropfen zu einer heterogenen nanoporösen Schicht mit durchschnittlicher Porengröße von 10 nm. Die halbleitenden Siliziumnanopartikel werden durch leitende gesinterte Goldnanopartikel elektrisch verbunden.

Die Zusammensetzung dieser nanoporösen Schicht ist eine Matrix aus halbleitenden Siliziumnanokristallen, die mit einander elektrisch durch die Goldnanopartikel verbunden sind. Diese Schicht kann für die Anwendungen in Solarzellen benutzt werden. Mit diesem Verfahren kann man sehr dünne gedruckte Schichten von lichtempfindichen Elementen mit einer niedrigen Sintertemperatur herstellen. Die Nanoerlauben den Durchgang des Lichtes zu tieferen Siliziumkristallen und dadurch größere Lichtabsorption und entsprechend höhere Effizienz der Solarzelle.

Kisner, A.; Lenk, S.; Mayer, D.; Mourzina, Y.; Offenhäusser, A. J. Phys. Chem. C 2009, 113, 20143-20147.

Ausführungsbeispiel mit verschiedener chemischer Zusammensetzung der Nanopartikel:
Zwei verschiedene Arten von Nanopartikeln werden für die Herstellung einer nanoporösen Schicht ausgewählt. Eine Art von Nanopartikeln sind runde homogene leitende Goldnanopartikel mit einem durchschnittlichen Durchmesser von 5 nm und einer Schutzschicht aus Zitrat gekauft von http://www.nanopartz.com (Produkt # A11C-5).
Die zweite Art von Nanopartikeln sind runde homogene leitende Silbernanopartikel mit einem durchschnittlichen Durchmesser von 100 nm gekauft von http://www.sigmaaldrich.com (Produkt # 730777).

Eine Mischung von diesen zwei verschieden Arten von Nanopartikeln wird vorbereitet. Deionisiertes Wasser wird als Lösungsmittel in dieser Mischung benutzt. Der prozentuelle Gewichtsanteil von runden Goldnanopartikeln (5 nm) mit dem Schmelzpunkt Sₘ₁ von 180° C in der Mischung ist 0,25%. Der prozentuelle Gewichtsanteil von runden Silbernanopartikeln (100 nm) mit dem Schmelzpunkt Sₘ₂ von 300° C in der Mischung ist 5%.

Ein Substrat mit löslicher Opferschicht wird vorbereitet. Z.B. ein 4-Zoll Glassubstrat (Borosilikat-Wafer, Collegewafers Inc.) wird durch PVD (physical vapor deposition) Aufdampf-Verfahren mit einer 10 nm Chrom-Schicht beschichtet.
Die vorbereitete Nanopartikel-Mischung wird auf das mit dem Chrom beschichtete Glassubstrat durch Spincoating aufgebracht. Dazu braucht man ungefähr 10 mL von der Nanopartikelnlösung, die mit einer Drehzahl von 3000 für 45 Sekunden aufgespinnt werden kann. Das Lösungsmittel wird bei Raumtemperatur innerhalb von 5 Minuten verdampft.
Das mit der Nanopartikelmischung beschichtete, ausgetrocknete Glassubstrat wird auf der Heizplatte bei 230° C für 5 Minuten erhitzt um die Nanopartikel zu sintern.

Die dadurch entstandene nanoporöse Schicht wird mit Rasterelektronmikroskopie überprüft. Durch das Sintern ändert sich der getrocknete Tropfen zu einer heterogenen nanoporösen Schicht mit durchschnittlicher Porengröße von 10 nm. Die leitenden Silbernanopartikel werden durch leitende gesinterte Goldnanopartikel elektrisch verbunden.

Die Zusammensetzung dieser nanoporösen Schicht ist eine Matrix aus leitenden Silbernanopartikeln, die miteinander elektrisch durch die Goldnanopartikel verbunden sind. Diese Schicht kann für die Anwendungen in aktiven nanoporösen Membranen benutzt werden.

## Patentansprüche

1. Verfahren zur Herstellung einer nanoporösen Schicht auf einem Substrat,
mit den Schritten:
a) Auswahl zweier Partikelsorten A und B mit unterschiedlichem Schmelzpunkt;
b) Suspendieren beider Partikelsorten A und B in einem Lösungsmittel, wobei die Partikel A und B in einem Gewichtsverhältnis von A:B von 5:1 bis 1000000:1 wt/wt zueinander eingestellt werden;
c) Aufbringen der Suspension enthaltend A und B auf dem Substrat;
d) Kontrolliertes Schmelzen der Partikel mit niedrigerem Schmelzpunkt S_{mB}, ohne den Schmelzpunkt S_{mA} der bei höherer Temperatur schmelzenden Partikel zu erreichen;
e) Überprüfen der Oberfläche der gebildeten nanoporösen Schicht auf dem Substrat.

2. Verfahren nach Anspruch 1,
**gekennzeichnet durch**
Wahl von Partikeln der Sorten A und B, bei denen der Unterschied im Schmelzpunkt S_{mA} und S_{mB} durch die verschiedene chemische Zusammensetzung der Materialien der Partikel A und B bedingt wird.

3. Verfahren nach einem der vorhergehenden Ansprüche,
**gekennzeichnet durch**
Wahl von Partikeln der Sorten A und B, bei denen der Unterschied im Schmelzpunkt S_{mA} und S_{mB} durch die verschiedene Größe der Partikel von A und B bedingt wird.

4. Verfahren nach einem der vorhergehenden Ansprüche,
**gekennzeichnet durch**
Wahl von Partikeln der Sorten A und B, bei denen der Unterschied im Schmelzpunkt S_{mA} und S_{mB} durch die verschiedene Form der Partikel von A und B bedingt wird.

5. Verfahren nach einem der vorhergehenden Ansprüche,
wobei Partikel A und B ausgewählt werden, bei denen der Unterschied im Schmelzpunkt S_{mA} und S_{mB} wenigstens 1K beträgt.

6. Verfahren nach einem der vorhergehenden Ansprüche,
**dadurch gekennzeichnet, dass**
nach der Überprüfung der nanoporösen Schicht die Schritte a) bis e) von Anspruch 1 erneut durchgeführt werden.

7. Verfahren nach einem der vorhergehenden Ansprüche,
bei dem
nach oder während der Herstellung der nanoporösen Schicht eine Funktionalisierung der nanoporösen Schicht mittels immunologisch wirksamen Antikörpern oder Antigenen erfolgt.

8. Verfahren nach vorhergehendem Anspruch,
**dadurch gekennzeichnet, dass**
nach der Funktionalisierung der nanoporösen Schicht diese kontaktiert wird, um elektrische Signale nach einer Bindung der immunologisch wirksamen Antigene oder Antikörper mit einem Analyten abzuleiten und nachzuweisen.

9. Nanoporöse Schicht hergestellt nach einem Verfahren der vorhergehenden Ansprüche,
**dadurch gekennzeichnet, dass**
die nanoporöse Schicht eine gleichmäßige Verteilung der Poren aufweist.

10. Nanoporöse Schicht nach vorherigem Anspruch,
**gekennzeichnet durch**
eine Beladung der Poren mit Antigenen oder Antikörpern.

## Claims

1. Method for producing a nanoporous layer on a substrate, with the steps:
a) Selecting two particle types A and B with different melting points;
b) Suspending both particle types A and B in a solvent, in which the particles A and B are adjusted in an A:B weight ratio of 5:1 to 1000000:1 wt/wt to each other;
c) Applying the suspension containing A and B to the substrate;
d) Controlled melting of the particles with lower melting point S_{mB}, without reaching the melting point S_{mA} of the particles melting at a higher temperature;
e) Checking the surface of the nanoporous layer formed on the substrate.

2. Method according to claim 1,
**characterised by**
selecting particles of types A and B, in which the difference in melting points S_{mA} and S_{mB} is determined by the different chemical composition of the materials of particles A and B.

3. Method according to one of the previous claims,
**characterised by**
selecting particles of types A and B, in which the difference in melting points S_{mA} and S_{mB} is determined by the different particle sizes of A and B.

4. Method according to one of the previous claims,
**characterised by**
selecting particles of types A and B, in which the difference in melting points S_{mA} and S_{mB} is determined by the different shapes of particles A and B.

5. Method according to one of the previous claims,
**in which** particles A and B are selected, in which the difference in melting points S_{mA} and S_{mB} is at least 1K.

6. Method according to one of the previous claims,
**characterised in that**
after examining the nanoporous layer steps a) to e) of claim 1 are carried out again.

7. Method according to one of the previous claims,
in which
after or during production of the nanoporous layer functionalisation of the nanoporous layer is carried out by means of immunologically effective antibodies or antigens.

8. Method according to the previous claim,
**characterised in that**
after functionalisation of the nanoporous layer it is contacted in order to conduct and detect electrical signals after the immunologically effective antigens or antibodies bind with an analyte.

9. Nanoporous layer produced according to a method of the previous claims,
**characterised in that**
the nanoporous layer has an even distribution of pores.

10. Nanoporous layer according to the previous claim,
**characterised by**
loading the pores with antigens or antibodies.

## Revendications

1. Procédé de fabrication d'une couche nanoporeuse sur un substrat, avec les étapes de :
a) sélection de deux sortes de particules A et B ayant un point de fusion différent ;
b) suspension des deux sortes de particules A et B dans un solvant, dans lequel les particules A et B sont ajustées l'une par rapport à l'autre en un rapport en poids de A:B de 5:1 à 1 000 000:1 en poids/poids ;
c) dépôt de la suspension contenant A et B sur le substrat ;
d) fusion contrôlée des particules avec un point de fusion plus faible S_{mB}, sans parvenir au point de fusion S_{mA} des particules fondant à température plus élevée ;
e) contrôle de la surface de la couche nanoporeuse formée sur le substrat.

2. Procédé selon la revendication 1,
**caractérisé par**
la sélection de particules des sortes A et B, dans lesquelles la différence de point de fusion S_{mA} et S_{mB} est conditionnée par la composition chimique différente des matériaux des particules A et B.

3. Procédé selon l'une des revendications précédentes,
**caractérisé par**
la sélection de particules des sortes A et B, dans lesquelles la différence de point de fusion S_{mA} et S_{mB} est conditionnée par la taille différente des particules A et B.

4. Procédé selon l'une des revendications précédentes,
**caractérisé par**
la sélection de particules des sortes A et B, dans lesquelles la différence de point de fusion S_{mA} et S_{mB} est conditionnée par la forme différente des particules A et B.

5. Procédé selon l'une des revendications précédentes,
dans lequel des particules A et B sont choisies, dans lesquelles la différence de point de fusion S_{mA} et S_{mB} est au moins de 1 K.

6. Procédé selon l'une des revendications précédentes,
**caractérisé en ce que**
après le contrôle de la couche nanoporeuse, les étapes a) à e) de la revendication 1 sont réalisées de nouveau.

7. Procédé selon l'une des revendications précédentes,
dans lequel
après ou pendant la fabrication de la couche nanoporeuse, une fonctionnalisation de la couche nanoporeuse s'effectue au moyen d'anticorps ou d'antigènes immunologiquement actifs.

8. Procédé selon la revendication précédente,
**caractérisé en ce que**
après la fonctionnalisation de la couche nanoporeuse, celle-ci est mise en contact pour dévier et détecter les signaux électriques après une liaison des antigènes ou anticorps immunologiquement actifs avec un analyte.

9. Couche nanoporeuse fabriquée d'après un procédé des revendications précédentes,
**caractérisée en ce que**
la couche nanoporeuse présente une distribution régulière de pores.

10. Couche nanoporeuse selon la revendication précédente,
**caractérisée par**
un chargement des pores avec des antigènes ou des anticorps.
